Europäisches Patentamt

European Patent Office

Office européen des brevets

(11) Numéro de publication: **0 322 264**
**A1**

(12) **DEMANDE DE BREVET EUROPEEN**

(21) Numéro de dépôt: 88402926.5

(22) Date de dépôt: 22.11.88

(51) Int. Cl.⁴: **A 01 J 7/00**
G 01 N 1/20, G 01 F 23/26,
G 01 F 5/00

(30) Priorité: 23.11.87 FR 8716208

(43) Date de publication de la demande:
28.06.89 Bulletin 89/26

(84) Etats contractants désignés:
BE CH DE ES GB IT LI NL

(71) Demandeur: Savoyet, Jean-Louis Paul Jules
La Mayrie
F-38770 La Motte d'Aveillans (FR)

(72) Inventeur: Savoyet, Jean-Louis Paul Jules
La Mayrie
F-38770 La Motte d'Aveillans (FR)

(74) Mandataire: Casalonga, Axel et al
BUREAU D.A. CASALONGA - JOSSE Morassistrasse 8
D-8000 Munich 5 (DE)

Le titre de l'invention a été modifié (Directives relatives à l'examen pratiqué à l'OEB, A-III, 7.3)

(54) **Dispositif pour le prélèvement d'un échantillon et la mesure du débit d'un liquide en circulation constante ou pulsée.**

(57) Dispositif de prélèvement d'un échautillon et de mesure du débit d'un liquide comprenant une surface supérieure conique convexe (9) du cône mâle ayant un angle au sommet légèrement plus important que la surface inférieure conique concave (10) du cône femelle (5), de manière à ménager entre les deux une chambre (11) dont l'épaisseur va en décroissant du centre vers la périphérie, cette épaisseur étant en outre maintenue à une valeur minimale grâce à des entretoises (12) portées par l'un des cônes.

Des moyens de prélèvement comprennent des feutes verticales (21), pratiquées dans la paroi de la chambre cylindrique (2) prolongées vers l'intérieur par une pièce de prélèvement (22) et dirigeant le flux prélevé vers le réceptacle (28).

Application principale à l'industrie laitière.

FIG.1

## Description

### Dispositif pour le prélèvement et la mesure du débit d'un liquide en circulation constante ou pulsée.

On a décrit dans le brevet français 2548360, au nom du demandeur, un dispositif de prélèvement et de mesure du débit d'un liquide en circulation constante ou pulsée, notamment d'un liquide moussant tel que du lait, ce dispositif comportant un moyen pour réaliser un écoulement laminaire cylindrique du débit total, un moyen pour prélever une fraction précise et constante de cet écoulement laminaire et l'accumuler dans un réceptacle d'échantillon, ainsi qu'un moyen pour recevoir et évacuer le reste de l'écoulement laminaire, et un moyen électrique pour mesurer en temps réel directement dans le réceptacle la variation en fonction du temps du volume de l'échantillon, sans tenir compte du volume éventuel de la mousse, et en déduire la variation en fonction du temps du volume de l'écoulement total.

En particulier, dans un mode de réalisation préféré, l'écoulement laminaire cylindrique était obtenu à partir de l'écoulement d'arrivée en l'épanouissant entre un cône femelle supérieur fixe, en forme d'entonnoir inversé, et un cône mâle inférieur mobile, dont les génératrices étaient parallèles à celles du cône femelle, et qui était monté sur ressort à partir d'une tige coulissante inférieure pour venir s'appliquer à une faible distance à l'intérieur du cône femelle.

Par ailleurs, le prélèvement proportionnel était obtenu par des ouvertures percées obliquement dans la paroi le long de laquelle et à l'intérieur de laquelle se produisait l'écoulement laminaire cylindrique. Enfin, la mesure de débit insensible au volume de mousse était obtenue par une mesure capacitive entre une électrode cylindrique centrale et la paroi cylindrique extérieure du réceptacle.

Le but de l'invention est d'apporter des perfectionnements à ces divers moyens de mise en écoulement laminaire, de prélèvement et de mesure en vue de rendre l'appareil plus précis et plus constant.

Le premier perfectionnement consiste à choisir pour le cône mâle inférieur mobile un angle au sommet de sa surface convexe légèrement plus grand que l'angle au sommet de la surface conique intérieure concave du cône femelle, afin de ménager entre les deux une chambre dont l'épaisseur décroît du centre vers la périphérie, au fur et à mesure que la circonférence de l'écoulement augmente. En outre, ce cône mâle est monté par l'intermédiaire d'une tige dépassant à la partie supérieure et coulissant non pas dans la base de la chambre d'écoulement, mais dans la partie supérieure du cône femelle pour un meilleur centrage, cette tige étant immobilisée en translation et éventuellement en rotation par rapport au cône mâle et au cône femelle, le ressort de sollicitation étant également placé en position supérieure.

Un deuxième perfectionnement consiste à remplacer les simples ouvertures de prélèvement disposées dans la paroi par des fentes verticales prolongées vers l'intérieur par des parois parallèles rapprochées dont le bord supérieur est affûté en forme de couteau pour un prélèvement plus précis. Ces couteaux sont de préférence amovibles et montés par simple ressort.

Un troisième perfectionnement consiste à disposer entre la paroi extérieure du réceptacle et l'électrode centrale de mesure, un tube métallique laissant subsister à son extrémité supérieure un passage d'équilibrage des pressions, et à son extrémité inférieure une ou plusieurs ouvertures de très faible hauteur laissant passer le liquide mesuré en s'opposant au passage de la mousse, la mesure ayant alors lieu entre l'électrode centrale, qui est une simple tige métallique recouverte d'un isolant diélectrique tel que du polytétrafluoréthylène, et ledit tube, et non plus entre l'électrode et la paroi extérieure.

D'autres particularités de l'invention apparaîtront dans la description qui va suivre d'un mode de réalisation pris comme exemple et représenté sur le dessin annexé, sur lequel :

la figure 1 est une coupe axiale du dispositif; et

la figure 2 représente, à plus grande échelle, une vue extérieure selon II d'un des couteaux de prélèvement.

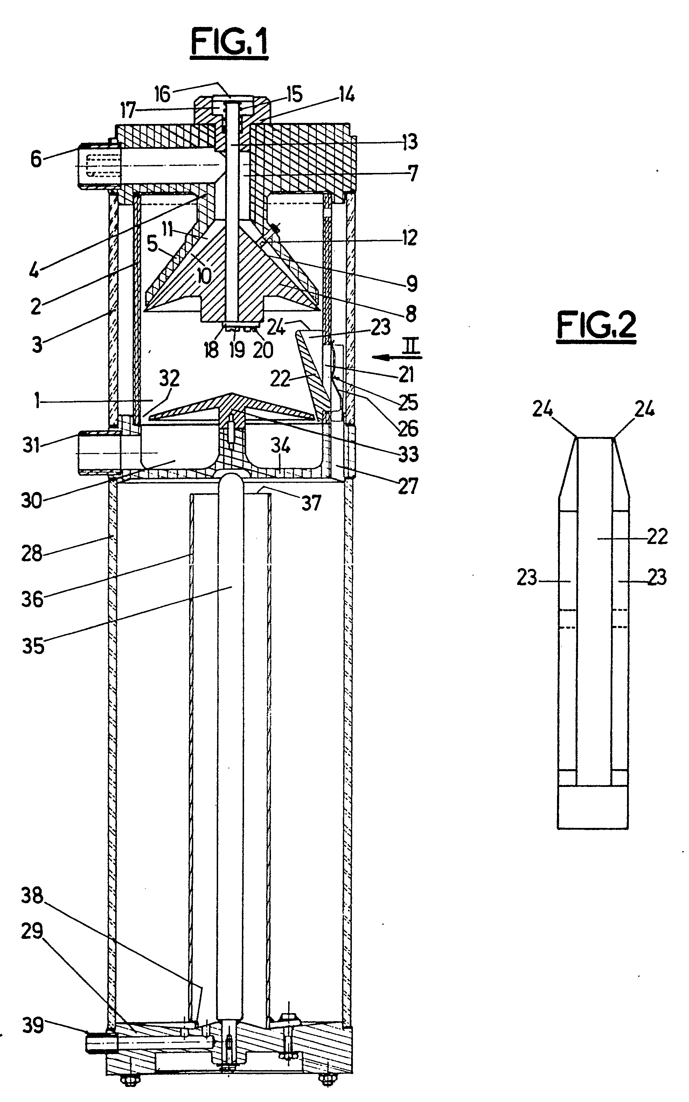

On voit sur la figure 1 la chambre de prélèvement 1, avec sa paroi d'écoulement 2 et sa paroi extérieure 3, et la partie supérieure fixe 4 incluant le cône femelle 5.

L'arrivée du liquide se fait par l'embout 6 et aboutit dans l'axe du cône femelle en 7 avant de s'épanouir entre le cône femelle et le cône mâle 8, lequel, conformément au présent certificat d'addition, a un angle au sommet plus grand que celui du cône femelle 5, de manière à former entre la surface extérieure conique convexe 9 du cône mâle et la surface intérieure conique concave 10 du cône femelle, une chambre 11 dont l'épaisseur va en décroissant du centre vers la périphérie, au fur et à mesure que l'écoulement du liquide arrivant en 7 s'épanouit selon des circonférences de diamètre croissant. L'épaisseur minimale de cet écoulement à la périphérie de l'ensemble est définie par des entretoises 12, par exemple au nombre de trois, situées à 120° de l'axe, et qui sont par exemple fixées dans le cône femelle 5 pour servir de butée au cône mâle 8.

Le cône mâle 8 est monté par l'intermédiaire d'une tige axiale 13 qui dépasse à sa partie supérieure et coulisse à son extrémité supérieure dans une pièce 14 fixée à l'extrémité supérieure de la partie fixe 4. Le ressort de rappel 15 est disposé à l'intérieur de cette pièce 14 et autour de la tige 13, et s'appuie sur un bouton d'extrémité 16, solidaire en translation et en rotation de l'extrémité supérieure de la tige 13, et de préférence immobilisé également en rotation par rapport à la pièce 14. Le bouton 13 est par exemple de forme rectangulaire et pénètre dans un fraisage rectangulaire 17 pratiqué diamétralement dans la partie supérieure de la pièce 14. Par ailleurs, le cône mâle 8 est immobilisé en translation, et est de préférence également en rotation par rapport à l'extrémité inférieure de la tige 13 grâce à une barrette 18 fixée par deux vis 19 et 20, respectivement sur l'extrémité de la tige 13 et sur le cône 8.

De cette manière, la poussée du ressort 15 maitient constamment le cône mâle 8 en butée contre les entretoises 12. Toutefois, en cas d'engorgement, par exemple par la présence d'agglomérations de particules de crème, il est possible de dégager l'écoulement en appuyant simplement sur le bouton 16 à la partie supérieure de l'appareil.

Depuis la périphérie de l'ensemble des cônes mâle 8 et femelle 5, l'écoulement laminaire se poursuit contre la surface intérieure de l'enveloppe cylindrique 2 de la chambre d'écoulement 1. C'est là que s'opère le prélèvement de la fraction précise du débit total de cet écoulement. Comme dans le brevet principal, on utilise pour cela des ouvertures, de préférence au nombre de trois, réparties à 120° autour de l'axe de l'appareil pour rendre le prélèvement insensible aux petites imperfections de verticalité de l'appareil. Toutefois, chacune de ces ouvertures 21 est en forme de fente orientée parallèlement aux génératrices du cylindre 2 et prolongée vers l'intérieur de la chambre d'écoulement 1 par une pièce 22 dont la section horizontale est en forme de U, avec deux ailes latérales 23 dont le bord supérieur 24, sensiblement horizontal, est affûté comme représenté sur la figure 2 pour découper dans la lame de l'écoulement laminaire une largeur précise correspondant à l'écartement entre les deux arrêtes affûtées 24. L'ensemble de chacune de ces pièces 22 en forme de double couteau est monté dans l'une des fentes 21 depuis l'intérieur de la paroi 2, de préférence d'une manière amovible, par exemple au moyen d'une goupille 25 et d'un ressort 26. La forme et l'inclinaison de ces pièces 22 sont telles qu'elles dirigent le flux prélevé vers l'extérieur de la paroi 2.

Les fractions du débit ainsi recueillies par les trois couteaux 22 sont conduites dans l'intervalle entre les deux parois 2 et 3, et de là s'écoulent par une ou plusieurs ouvertures 27 dans le réceptacle inférieur, constitué par une paroi cylindrique 28 refermée à sa base par une pièce de fond 29.

Le reste de l'écoulement laminaire, qui constitue la majeure partie restant après ce prélèvement, s'écoule dans une chambre d'aspiration 30, en communication avec l'embout de sortie 31, cette chambre de communication étant à la base de la chambre d'écoulement 1 avec laquelle elle se raccorde, non plus par un simple orifice comme dans le brevet principal, mais par une fente circulaire continue 32 située entre la base de la paroi d'écoulement cylindrique 2 et un champignon central 33 à surface supérieure conique, ce champignon 33 étant fixé dans la pièce 34 située entre la chambre d'écoulement 1 et le réceptacle 28.

Dans l'axe de ce réceptacle cylindrique 28 est disposée une électrode de mesure 35, par exemple en acier inoxydable revêtu d'un diélectrique tel que du polytétrafluoréthylène, pour permettre la mesure capacitive de la hauteur du liquide accumulé dans le réceptacle 28. Toutefois, au lieu de procéder à cette mesure directement entre l'électrode centrale 35 et la paroi extérieure 28, comme dans le brevet principal, il est préférable de disposer un tube métallique 36 alentour de cette électrode centrale 35 pour constituer la deuxième électrode de mesure. De ce fait, la paroi extérieure 28 du réceptacle n'a plus besoin d'être métallique, et la mousse formée par l'écoulement du liquide reste cantonée à l'extérieur de la cellule de mesure constituée par les électrodes 35 et 36, en prévoyant pour cela un tube 36 dépourvu de toute ouverture, excepté son ouverture supérieure 37, qui débouche à très faible distance au-dessous de la pièce 34, et une ou plusieurs ouvertures inférieures 38 d'une hauteur très faible pour permettre le passage du liquide en empêchant le passage de la mousse. De cette manière, la mesure est encore plus précise et plus indépendante de la mousse que dans le dispositif selon le brevet principal.

Bien entendu, après l'opération de mesure, le liquide accumulé dans le réceptacle 28 est évacué par l'embout 39 de la pièce de fond 29 communiquant avec la base du réceptacle, aussi bien à l'extérieur qu'à l'intérieur du tube de mesure 36.

## Revendications

1. Dispositif de prélèvement et de mesure du débit d'un liquide, comportant une amenée centrale (7) du liquide s'épanouissant entre un cône femelle extérieur fixe (5) et un cône mâle intérieur mobile (8) pour produire un écoulement laminaire cylindrique à l'intérieur d'une enveloppe d'écoulement (2) comportant des orifices (21) assurant le prélèvement d'une fraction précise et constante de cet écoulement cylindrique recueillie dans un réceptacle (28), et une chambre d'aspiration (30) en communication avec la chambre d'écoulement pour recueillir et évacuer la partie non prélevée de l'écoulement, la mesure du niveau de la partie prélevée dans le réceptacle étant déterminée électriquement par mesure capacitive à l'aide d'une électrode centrale (35), caractérisé par le fait que la surface supérieure conique convexe (9) du cône mâle (8) a un angle au sommet légèrement plus important que la surface inférieure conique concave (10) du cône femelle (5), de manière à ménager entre les deux une chambre (11) dont l'épaisseur va en décroissant du centre vers la périphérie, cette épaisseur étant en outre maintenue à une valeur minimale grâce à des entretoises (12) portées par l'un des cônes.

2. Dispositif selon la revendication 1, caractérisé par le fait que le cône mâle (8) est solidaire au moins en translation d'une tige (13) dépassant à son extrémité supérieure et coulissant dans une pièce (14) solidaire de la partie supérieure (4) de l'appareil, cette tige étant solidaire au moins en translation d'un bouton (16) situé à son extrémité supérieure et repoussé axialement vers le haut par un ressort de rappel (15), appliquant le cône mâle (8) contre les entretoises (12), tout en permettant d'accroître temporairement l'épaisseur de la chambre (11) pour un dégagement par action manuelle sur le bouton (16).

3. Dispositif selon la revendication 2, caractérisé par le fait que la tige (13) est immobilisée également en rotation, à la fois par rapport au cône mâle (8) et par rapport au bouton (16), et que ce dernier est lui-même immobilisé en rotation par rapport à la pièce supérieure (14) dans laquelle il coulisse.

4. Dispositif selon une des revendications précédentes, caractérisé par le fait que chacune des ouvertures de prélèvement (21) pratiquée dans la paroi cylindrique (2) de la chambre d'écoulement (1) se prolonge vers l'intérieur par une pièce de prélèvement (22) à section sensiblement en U très étroite, dont les bords supérieurs (24) des ailes sont en forme de couteaux à arêtes affûtées parallèles et à un faible intervalle définissant la fraction prélevée, la forme et l'inclinaison de ces pièces dirigeant le flux prélevé vers l'extérieur de la paroi (2) de la chambre d'écoulement (1), et de là vers le réceptacle (28).

5. Dispositif selon une des revendications précédentes, caractérisé par le fait que l'orifice d'écoulement (32) entre la chambre d'écoulement (1) et la chambre d'aspiration (30) est constitué par une fente circulaire étroite (32) ménagée entre la paroi (2) de la chambre d'écoulement et un champignon central (33) à surface supérieure convexe solidaire de la base (34) de la chambre d'aspiration.

6. Dispositif selon une des revendications précédentes, caractérisé par le fait que l'électrode centrale de mesure (35) est revêtue d'un diélectrique tel que du polytétrafluoréthylène, et entourée par un tube métallique de mesure (36) dépourvu d'orifice, excepté son orifice supérieur (37) situé très près de la paroi supérieure (34) du réceptacle (28) et servant à l'équilibrage des pressions, et un ou plusieurs orifices inférieurs (38) situés très près de la pièce de fond (29) du réceptacle (28), la hauteur de ces ouvertures (38) étant suffisante pour permettre le passage du liquide et insuffisante pour permettre le passage de la mousse éventuellement formée par l'écoulement, la mesure capacitive du niveau du liquide étant alors effectuée entre l'électrode centrale (35) et ledit tube de mesure (36).

## FIG.1

## FIG.2

## DOCUMENTS CONSIDERES COMME PERTINENTS

| Catégorie | Citation du document avec indication, en cas dè besoin, des parties pertinentes | Revendication concernée | CLASSEMENT DE LA DEMANDE (Int. Cl.4) |
|---|---|---|---|
| Y,D | EP-A-0 132 209 (JEAN-LOUIS SAVOYET) * Page 2, ligne 19 – page 3, ligne 10; page 3, lignes 16-21; page 7, lignes 14-18; figures 3,4 * | 1 | A 01 J 7/00 G 01 N 1/20 G 01 F 23/26 G 01 F 5/00 |
| A | | 5 | |
| Y | US-A-3 600 944 (W.D.J. HUTCHINGS) * Colonne 4, lignes 21-27; figure 3 * | 1 | |
| A | EP-A-0 047 555 (N.V. VERENIGDE INSTRUMENTEN ENRAF-NONIUS) * Résumé; page 11, lignes 11-14,19-25; figure 10 * | 1-3 | |
| A | US-A-3 045 493 (T.L. SEABORNE) * Colonne 1, lignes 9-15; colonne 1, ligne 65 – colonne 2, ligne 3; figure 1 * | 1,4 | |
| A | US-A-4 346 596 (DIAMANT et al.) * Résumé: colonne 2, lignes 39-43; colonne 3, lignes 24-30; figure 1 * | 1,6 | |
| A | GB-A-1 139 460 (T.L. SEABORNE) * Page 1, lignes 54-82; figure 1 * | 1 | **DOMAINES TECHNIQUES RECHERCHES (Int. Cl.4)** A 01 J G 01 N G 01 F |
| A | GB-A-1 314 016 (UNITED KINGDOM ATOMIC ENERGY AUTHORITY) * Page 2, lignes 17-22,39-45,49-52,82-85; figure * | 1 | |
| A | EP-A-0 012 734 (BOLIDEN AB) * Résumé; figures 1,2 * | 4 | |

Le présent rapport a été établi pour toutes les revendications

| Lieu de la recherche | Date d'achèvement de la recherche | Examinateur |
|---|---|---|
| LA HAYE | 21-02-1989 | GANCI P.A. |